# EUROPEAN PATENT APPLICATION

(11) **EP 3 530 177 A1**
(43) Date of publication of application: **28.08.2019**
(21) Application number: 18158743.7
(22) Date of filing: 27.02.2018
(51) Int. Cl.: A61B 5/00, A61B 5/055

(54) **POSITIONING OF AN ANATOMICAL STRUCTURE OF INTEREST**

(71) Applicant: Koninklijke Philips N.V., 5656 AE Eindhoven (NL)
(72) Inventor: WARNER, Lizette, 5656 AE Eindhoven (NL); WEISS, Steffen, 5656 AE Eindhoven (NL); SCHUBERT, Gerald, 5656 AE Eindhoven (NL)
(74) Representative: de Haan, Poul Erik

(57) **Abstract**

The invention relates to an antenna element (100) configured for positioning an anatomical structure of interest of a subject (218) in a position defined by the antenna element (100) for acquiring the magnetic resonance imaging data (244), to a positioning device (150) for positioning the anatomical structure of interest in the defined position on a support (348) of a medical radiation delivery system (300), to a fixating element (130) for fixating the anatomical structure of interest in the defined position on the antenna element (100) as well as on the positioning device (150).

## Description

### FIELD OF THE INVENTION

The invention relates to magnetic resonance imaging and radiation delivery, in particular to positioning of an anatomical structure for magnetic resonance imaging and radiation delivery.

### BACKGROUND OF THE INVENTION

In order to ensure a safe, precise and effective delivery of radiation to a target area comprised by an anatomical structure of interest, e.g. in order to kill malignant cells, a careful planning of radiation doses and determining of target area may be necessary in order to maximize radiation delivered to the target area, while minimizing the impact of the radiation delivery on adjacent tissue comprised. This planning may also be referred to as radiation therapy simulation. For example, computed tomography (CT) images or magnetic resonance imaging (MRI) images may be utilized for defining target areas and radiation doses to be delivered. In order to ensure the same position of the anatomical structure of interest for imaging and radiation delivery, the position of the anatomical structure of interest intended for the radiation delivery may be mimicked during imaging. For radiation delivery in general a flat tabletop is used for supporting a subject with the anatomical structure of interest. To improve positioning, for example, a flat tabletop may be utilized for radiation therapy simulation as well, e.g. using MRI.

Thus, radiation therapy simulation using MRI may involve placing the subject on a support of the MRI system ensuring the same position as the one utilized for the actual radiation delivery. The support used for the simulation may be a flat tabletop equal to a flat tabletop used for radiation delivery in order to positioning the subject equally as for radiation delivery. The usage of a flat tabletop for both radiation therapy simulation and radiation delivery may facilitate the positioning as well as take into account position depending influences of the radiation delivery during radiation delivery, since the delivery may thus be executed with the subject in the same position as during simulation. However, positioning of the anatomical structure of interest directly on a flat tabletop for imaging may prevent antenna elements, also referred to as coils, from being arranged in the vicinity of the anatomical structure of interest. In particular, in case a posterior antenna element, i.e. an antenna element positioned on the posterior side of the subject, the distance between the anatomical structure of interest antenna element may be increased due to the tabletop. The increased distance of the antenna element may result in simulation imaging suffering from inferior image quality compared with magnetic resonance imaging using antenna elements which are positioned against the anatomical structure of interest, thus minimizing the distance between antenna element and anatomical structure of interest.

MRI may use specialized antenna element for acquiring precision MRI data. However, the geometry of such antenna elements may not allow a positioning of the anatomical structure of interest in the same position as for radiation delivery. For example, MRI brain imaging may be executed using a specialized antenna element in form of a head-coil, while for radiation therapy simulation no head-coil can be used, when the position of for the radiation delivery should be mimicked. Not being able to use the head-coil for radiation therapy simulation may compromise the image quality, especially in respect to geometrical accuracy and image intensity homogeneity. This may be a major disadvantage in possible MRI-only radiation therapy simulation, wherein simulation is solely based on MRI images.

### SUMMARY OF THE INVENTION

The invention provides for an antenna element for a magnetic resonance imaging system, a positioning device for a medical radiation delivery system, a fixating element for the antenna element as well as the positioning device, for a kit comprising the antenna element, the positioning device and the fixating element, a method for positioning an anatomical structure of interest and a method for generating the fixating element in the independent claims. Embodiments are given in the dependent claims.

In one aspect, the invention relates to an antenna element for a magnetic resonance imaging system. The antenna element is configured for acquiring magnetic resonance imaging data of an anatomical structure of interest of a subject. The antenna element comprises a curved antenna surface which is adapted at least partially to a geometrical form of the anatomical structure of interest and configured to force the anatomical structure of interest in a position defined by the antenna element for acquiring the magnetic resonance imaging data, when the anatomical structure of interest is positioned against the curved antenna surface.

The antenna element further comprises mounting means configured for mounting a fixating element on the antenna element and establishing a detachable connection between the antenna element and the fixating element.

The fixating element comprises a second curved surface which is adapted at least partially to the geometrical form of the anatomical structure of interest and configured to fixate the anatomical structure of interest in the defined position, when the second curved surface is positioned against the anatomical structure of interest and the fixating element is mounted on the antenna element establishing the detachable connection between the antenna element and the fixating element.

Embodiments may have the beneficial effect of providing an antenna element which enables a precise positioning of the anatomical structure of interest for magnetic resonance data acquisition. In addition, the antenna element provides mounting means for mounting the fixating element, like e.g. a face mask in case of an MRI head-coil, on the antenna element, thus fixating the anatomical structure of interest in the defined position. This way, it may be ensured that the MRI data is acquired with the anatomical structure in the defined position throughout data acquisition.

For example, MR imaging for MRI-only radiation therapy simulation regarding a head and/or neck of a patient may advantageously make use of a MRI head and/or neck coil, which may enable an optimized image quality.

According to embodiments, the curved antenna surface and/or the second curved surface may comprise concave and/or convex portions. Embodiments may have the beneficial effect that the curved surfaces may be efficiently adapted to the geometrical form of the anatomical structure of interest.

According to embodiments, the geometrical form of the anatomical structure of interest to which the curved antenna surface is adapted is defined by general geometrical features common to a plurality of anatomical structures of a plurality of subjects that are of the same type as the anatomical structure of interest.

Embodiments may have the beneficial effect of enabling a positioning of the patient in a fixed position in the MRI system, e.g. in the same position as in a medical radiation delivery system like an MRI guided LINAC, in a repeatable fashion without a requirement for designing individually customized antenna elements, but rather utilizing suitable standard MRI coil designs which are in addition provided with mounting means for mounting the fixating element. The proposed embodiments for generation of the fixating element may require only small modifications of a given MRI head-coil, like e.g. holes for alignment pins. An individual customization may be implemented by customizing the fixating element. Thus, the same antenna element may not only be reusable for positioning the same subject, but also to position a plurality of different subjects.

According to embodiments, the fixating element is mounted on the antenna element. Using a fixating element for magnetic resonance imaging, e.g. a face mask, may ensure a predefined patient posture and immobilized position during acquisition of magnetic resonance data. Embodiments may have the beneficial effect that the second curved surface may make contact with the anatomical structure of interest at a number of spatial locations to prevent motion of anatomical structure.

According to embodiments, the fixating element mounted on the antenna element may be an antenna element specific fixating element configured to precisely fit on the antenna element. According to embodiments, the fixating element mounted on the antenna element may be an antenna element configured to fit on the antenna element as well as on a positioning device for a medical radiation delivery system.

According to embodiments, the geometrical form of the anatomical structure of interest to which the second curved surface is adapted is defined by individual geometrical features of the anatomical structure of interest of the subject.

Embodiments may have the beneficial effect of implementing an individual customization by customizing the fixating element, while suitable standard MRI coil designs may be used for the antenna element. The second curved surface of the fixating element may be customized for an individual subject taking into account individual geometrical features of the anatomical structure of interest of the respective subject. Embodiments may have the beneficial effect of efficiently fixating anatomical structures of interest with different geometrical features, like e.g. different dimensions and/or shapes of heads, necks, knees or shoulders in a predefined position. For each of these anatomical structures of the same type, despite different dimensions and/or different geometrical shapes, the same antenna element may be used, while only the fixating element may have to be customized.

According to embodiments, the antenna element comprises receptions configured for receiving alignment means of the fixating element for aligning the fixating element on the antenna element, when the fixating element is mounted on the antenna element. According to embodiments, the mounting means comprises the receptions.

According to embodiments, the fixating element comprises a net structure, the net structure being configured to at least partially covering the anatomical structure of interest and to provide the second curved surface. Embodiments may have the beneficial effect that the fixating element may effectively and efficiently fixate the anatomical structure of interest in the predefined position. A net structure may in particular enable an individual adaption of the curved surface to individual geometrical features of an anatomical structure of interest.

According to embodiments, the fixating element comprises a face mask. According to embodiments, the face mask is provided in form of the net structure.

According to embodiments, the antenna element comprises one or more of the following: a head-coil, a neck coil, a knee coil and a shoulder coil. According to embodiments, the antenna element comprises two parts which are detachably connectable with each other, e.g. using additional mounting means. According to embodiments a first part of the antenna element provides the curved antenna surface against which the anatomical structure of interest is positioned and fixated using the fixating element mounted on the first part. The second part is mounted on the first part as well and may cover at least partially or even entirely the fixating element and the anatomical structure of interest. For example, the first part of the antenna element is a posterior part comprising a posterior coil, while the second part of the antenna element is an anterior part comprising anterior coil. According to embodiments, each of the two parts may comprise one or more additional coils. According to embodiments, the antenna element may comprise more than two parts which are detachably connectable with each other, e.g. using additional mounting means.

For example, the antenna element may be provided in form of a head and/or neck coil without full facial coverage. Thus, the fixating element, e.g. in form of a face mask, may easily be mounted on the respective antenna element, while the subject's head rests on a curved surface of the antenna element. According to further examples, the antenna element may be provided in form of a head and/or neck coil with a detachable facial coverage portion, e.g. comprising an anterior coil. The facial coverage portion maybe dismounted from the rest of the head and/or neck coil, the subject's head may be rested on the curved surface provided by the rest and the fixating element, e.g. in form of a face mask, may easily be mounted on the respective rest in order to fixate the head. Afterwards, the facial coverage portion may be remounted in order to ensure an optimal imaging quality.

According to embodiments, at least one of the antenna element and the fixating element comprises markers for a laser alignment of the antenna element with respect to the magnet resonance imaging system. Embodiments may have the beneficial effect of enabling a precise alignment. The alignment may e.g. be executed using a plurality of identical laser beam systems, e.g. two identical laser beam systems, that are themselves aligned with the MRI system or the medical radiation delivery system, like e.g. a LINAC system. This may allow a registration of the magnetic resonance imaging data with the medical radiation delivery system.

The workflow to use laser bridges to allow registration of the co-ordinate systems of the imaging modality used for MRI to the co-ordinate systems of the radiation delivery system may e.g. be used as follows: When an anterior part of a head-coil is removed, the laser beams may be directly visible on the fixating element of the antenna element, like e.g. an MRI mask. Respective laser markers may be applied to the mask. According to embodiments, their positions may in addition be included in the generation of a fixating element for the positioning device, e.g. an RT mask.

In another aspect, the invention relates to a positioning device for positioning an anatomical structure of interest of a subject on a support of a medical radiation delivery system according to a position defined by means of magnetic resonance imaging data of the anatomical structure of interest acquired using an antenna element of a magnetic resonance imaging system. A medical radiation delivery system may comprise a radiation source, like e.g. a linear accelerator (LINAC), used to generate high-energy radiation. An alternative radiation source may e.g. be provided using ⁶⁰CO radionuclides.

The positioning device comprises a first curved surface equal to an antenna surface of the antenna element. The first curved surface is adapted at least partially to a geometrical form of the anatomical structure of interest and configured to force the anatomical structure of interest in the defined position, when the anatomical structure of interest is positioned against the first curved surface.

The positioning device further comprises first mounting means configured for mounting the positioning device on the support and second mounting means configured for mounting a fixating element on the positioning device and for establishing a detachable connection between the positioning device and the fixating element.

The fixating element comprises a second curved surface. The second curved surface is adapted at least partially to the geometrical form of the anatomical structure of interest and configured to fixate the anatomical structure of interest in the defined position, when the second curved surface is positioned against the anatomical structure of interest and the fixating element is mounted on the positioning device establishing the detachable connection between the positioning device and the fixating element.

The positioning device is radiolucent for radiation delivered by the medical radiation delivery system. This may e.g. be achieved using materials like carbon fibre, glass fibre, low-density plastics, and rigid or semi-rigid foams.

Embodiments may have the beneficial effect of enabling to mimic the position of the anatomical structure of interest for acquiring the magnetic resonance imaging data in the medical radiation delivery system using the positioning device. The positioning device may for example be mounted on the support of the medical radiation delivery system provided in form of a flat tabletop. Mimicking the position for MRI imaging, when positioning the anatomical structure for radiation delivery may be opposite to current practice, wherein the radiation delivery position is mimicked in the MRI system for imaging and radiation therapy simulation. Embodiments may thus overcome the disadvantages of the current practice which may result in suboptimal coil positioning relative to the anatomical structure of interest and reduced image quality.

Embodiments may have the beneficial effect of ensuring that an anatomical structure of interest is forced to be positioned in a position defined by the MRI. The respective position may e.g. be defined as a position relative to a flat tabletop. According to embodiments the position may be defined by magnetic resonance positioning data. In order to acquire high quality magnetic resonance data, it has been proven advantageous to use an antenna element positioned in the vicinity of the anatomical structure of interest to be imaged. Thus, the smaller the distance between the respective anatomical structure of interest and the antenna element, the higher the quality of the acquired magnetic resonance data. Therefore, local antenna elements may be used, which are positioned next to the anatomical structure of interest to be imaged. Since the general geometrical form of anatomical structures of interest, i.e. the body shape of a human is not flat, respective antenna elements usually comprise curved surfaces against which an anatomical structure of interest can be closely positioned. Such a position of an anatomical structure of interest defined and supported by a curved surface in general differs from a position resulting from locating the anatomical structure of interest on a flat tabletop.

The positioning device, which may be mounted on a support, like a flat tabletop, of the medical radiation delivery system, may force the anatomical structure of interest into the predefined position, when being placed against the curved surface provided by the positioning device. Thus, the positioning device allows to mimic a position of the anatomical structure of interest defined by the MRI positioning data.

According to embodiments, the positioning device comprises a flat surface portion, wherein the distance of the curved surface of the positioning device from the flat surface as well as the orientation of the curved surface in space relative to the flat surface are equal to the distance of the curved surface of the antenna element as well as the orientation in space relative to a flat tabletop surface of a support of the magnetic resonance imaging system, when the antenna element is placed on the respective support according to its determined use.

Since the positioning device is radiolucent for the radiation delivered by the medical radiation delivery system, the positioning device does not interfere with the respective radiation. The respective radiation may for example be gamma rays or x-rays.

According to embodiments, the first and/or the second curved surfaces may comprise concave and/or convex portions. Embodiments may have the beneficial effect that the curved surfaces may be efficiently adapted to the geometrical form of the anatomical structure of interest.

According to embodiments, the geometrical form of the anatomical structure of interest to which the first curved surface is adapted is defined by general geometrical features common to a plurality of anatomical structures of a plurality of subjects that are of the same type as the anatomical structure of interest.

Embodiments may have the beneficial effect that the positioning device may be used for a plurality of subjects. The positioning device may for example be provided in form of a device with standardized dimensions and/or a standardized geometrical form.

According to embodiments, the medical radiation delivery system may be a standard radiotherapy (RT) system or an MRI guided RT system. According to embodiments, the medical radiation delivery system may comprise a support in form of a flat tabletop. The positioning device may comprise a flat surface which is positioned against the flat tabletop, when the positioning device is mounted on the support. According to embodiments, the positioning device may be utilized for positioning of the anatomical structure of interest in the medical radiation delivery system only and serve to mimic the MRI position of the anatomical structure. According to embodiments, the positioning device may be provided in form of a patient cradle.

According to embodiments, the fixating element is mounted on the positioning device.

Embodiments may have the beneficial effect that the positioning device in combination with the fixating element mounted on the positioning device may not only allow to force the anatomical structure of interest in the predefined position, but also to fixate the anatomical structure of interest in the predefined position. The fixating element may further ensure that the anatomical structure of interest is located in the defined position with a defined orientation. The anatomical structure of interest may in particular be prevented from being twisted in the respective position as well as from being removed from the defined position. According to embodiments the fixating element may be configured to be placed as a cover over the curved surface of the positioning device, wherein the curved surface of the fixating element faces the curved surface of the positioning device.

According to embodiments, the fixating element mounted on the positioning device may be a positioning device specific fixating element configured to precisely fit on the positioning device. According to embodiments, the fixating element mounted on the positioning device may be an antenna element configured to fit on the positioning device as well as on the antenna element for the magnetic resonance imaging system.

According to embodiments, the mounting means comprises an adapter detachably connected to the positioning device and configured for holding the positioning device on the support of the medical radiation delivery system. According to embodiments, the adapter is further configured to be detachably connected with the antenna element and for holding the antenna element on a support of the magnetic resonance imaging system.

Embodiments may have the beneficial effect of providing an adaptor which may be used for mounting the positioning device on a support of the medical radiation delivery system as well as for mounting the antenna element on a support of the magnetic resonance imaging system.

According to embodiments, the positioning device comprises receptions configured for receiving alignment means of the fixating element for aligning the fixating element on the positioning device, when the fixating element is mounted on the positioning device.

According to embodiments, the receptions of the positioning device are equal to the receptions of the antenna element. According to embodiments, the second mounting means comprises the receptions.

According to embodiments, at least one of the positioning device and the fixating element comprises markers for a laser alignment of the positioning device with respect to the medical radiation delivery system. Embodiments may have the beneficial effect of enabling a precise positioning using the laser markers.

According to embodiments, the second mounting means of the positioning device is equal to mounting means comprised by the antenna element. The mounting means of the antenna element is configured for mounting the fixating element on the antenna element and establishing a detachable connection between the antenna element and the fixating element. The second curved surface is further configured to fixate the anatomical structure of interest in the position defined by the antenna element, when the anatomical structure of interest is positioned against the antenna surface.

According the embodiments, one or more parts of the positioning device are radiolucent for radiation delivered by the medical radiation delivery system. According to embodiments the entire positioning device is radiolucent for radiation delivered by the medical radiation delivery system. According to embodiments, the positioning device comprises is at least one of the following: MRI and CT visible. According the embodiments, the positioning device comprises at least one of the following: MRI and CT markers.

In another aspect, the invention relates to a fixating element for fixating an anatomical structure of interest of a subject positioned in a position against a curved antenna surface of an antenna element of a magnetic resonance imaging system defined for acquiring magnetic resonance imaging data of the anatomical structure of interest and in the same position against a first curved surface of a positioning device of a medical radiation delivery system. The fixating element comprises mounting means configured for mounting the fixating element on the antenna element as well as on the positioning device and establishing a detachable connection between the fixating element and the antenna element as well as between the fixating element and the positioning device.

The fixating element further comprises a second curved surface adapted at least partially to the geometrical form of the anatomical structure of interest and configured to fixate the anatomical structure of interest in the defined position, when the second curved surface is positioned against the anatomical structure of interest and the fixating element is mounted on one of the following: the antenna element or the positioning device.

Embodiments may have the beneficial effect of that the fixating element may ensure a fixation of the anatomical structure of interest not only for radiation delivery, wherein a constant positioning is important for safety reasons, but also for MRI data acquisition in order the acquire high quality MRI images for determining e.g. target areas and organs at risk.

The fixating element, e.g. in form of a face mask, may be used for MRI to immobilize the anatomical structure of interest for MRI data acquisition as well as for radiation delivery in exactly the same posture. The fixating element be made from a flexible net structure that it fitted closely to the shape of the anatomical structure of interest, like e.g. a subject's head and then hardened. Generation of the fixating element may e.g. take about 30 min. The flexible net structure may e.g. be made from a thermoplastic.

Using MRI data for radiation therapy simulation may allow a detailed, high quality delineation of organs at risk based on the MRI data acquired using a magnetic resonance imaging system. Using MRI-only radiation therapy simulation may avoid introducing geometrical errors due to image registration. According to alternative embodiments, CT imaging may be used in addition to MRI. According to embodiments, the positing device may be used to position the anatomical structure of interest for CT imaging, i.e. the positing device may be used to position the anatomical structure of interest on a support, like a flat tabletop of a CT imaging system.

Currently, for MRI arrangements with a posterior coil located on the posterior side of the patient, which may be located e.g. inside or beneath a patient's support in form of a flat tabletop, an anterior coil located on the anterior side of the patient, as well as a plurality of, e.g. two, flexible coils may be used to provide as many coil elements as near as possible next to an anatomical structure of interest, like e.g. a head, and still be compatible with the position on a flat support of a medical radiation delivery system. Such a coil arrangement however maybe suboptimal in SNR and image quality, patient comfort, and workflow. Flexible coils refer to coils with a focus not fixed on a specific anatomical region of interest. Flexible coils may rather can be placed next to any part of a subject's anatomy.

Embodiments may have the beneficial effect of using an antenna element, like e.g. a head-coil, comprising a plurality of coil elements arranged at predefined relative positions to each other which are optimized in view of SNR as well as image quality. Thus, using the antenna element no additional adjusting of the coils comprised by the antenna element may be required.

According to embodiments, a fixating element, e.g. in form of a face mask, is provided and may be mounted on a posterior part of an antenna element, like e.g. an MRI head-coil. The fixating element may be used in combination with an antenna element comprising in addition an anterior part mounted on the posterior part, such that the fixating element is located between the two parts. Later on, the same fixating element or a second, equal fixating element may be used as an RT mask. The RT mask in combination with a positioning device may allow to fixate the anatomical structure of interest on a flat support table of a medical radiation delivery system in the same position, i.e. posture, as defined by the MRI mask and MRI coil during MRI.

According to embodiments, the fixating element further comprises alignment means for aligning the fixating element on the antenna element as well as on the positioning device. Embodiments may have the beneficial effect that by the alignment means, it may be ensured that the fixating element is aligned according to a predefined orientation on the positioning device.

According to embodiments, the alignment means in addition is configured for mounting the fixating element on the antenna element as well as on the positioning device.

Embodiments may have the beneficial effect that the mounting elements for mounting the fixating element on the positioning device may in addition ensure the alignment of the fixating element with a predefined orientation relative to the positioning device. Embodiments may have the beneficial effect that the fixating element may be configured to be mounted on the antenna element as well. In particular, it may be mounted on the antenna element such that the position fixated by the fixating element, when being mounted on the antenna element, is equal to the predefined position.

In other words, the alignment means may provide mounting means for mounting the fixating elements on the antenna element as well as on the positioning device. Embodiments may have the beneficial effect of enabling usage of the same fixating element, e.g. face mask, for fixating the anatomical structure of interest in the same position in the MRI system as well as in the medical radiation delivery system.

According the embodiments, the fixating element is radiolucent for radiation delivered by the medical radiation delivery system. According the embodiments, one or more parts of the fixating element are radiolucent for radiation delivered by the medical radiation delivery system. According to embodiments the entire fixating element is radiolucent for radiation delivered by the medical radiation delivery system. According to embodiments, the fixating element is at least one of the following: MRI and CT invisible. According to embodiments, the fixating element comprises is at least one of the following: MRI and CT visible. According the embodiments, the fixating element comprises at least one of the following: MRI and CT markers.

In another aspect, the invention relates to a kit comprising the antenna element of any of the previous embodiments for a magnetic resonance imaging system. The antenna element is configured for positioning the anatomical structure of interest of a subject in a position defined by the antenna element for acquiring magnetic resonance imaging data of the anatomical structure of interest and for acquiring the magnetic resonance imaging data. The kit further comprises the positioning device of any of the previous embodiments for positioning the anatomical structure of interest on a support of a medical radiation delivery system according to the position defined by the antenna element. Furthermore, the kit comprises the fixating element of any of the previous embodiments for fixating the anatomical structure of interest in the defined position against the antenna element as well as against the positioning device.

In another aspect, the invention relates to a method for positioning an anatomical structure of interest of a subject on a support of a medical radiation delivery system according to a position defined by an antenna element of a magnetic resonance imaging system. The method comprising defining the position using the antenna element for acquiring magnetic resonance imaging data of the anatomical structure of interest.

The anatomical structure of interest is positioned against a curved antenna surface of the antenna element during data acquisition. The curved antenna surface is at least partially adapted to a geometrical form of the anatomical structure of interest and configured to force the anatomical structure of interest in the defined position, when the anatomical structure of interest is positioned against the curved antenna surface. The anatomical structure of interest is positioned on the support of the medical radiation delivery system according to the position defined by the antenna element using a positioning device. The anatomical structure of interest is positioned against a first curved surface of the positioning device which is equal to the curved antenna surface.

The anatomical structure of interest is fixated in the defined position against the positioning device using a fixating element. The fixating element comprises a second curved surface and is mounted on the positioning device establishing a detachable connection between the positioning device and the fixating element. The second curved surface is adapted at least partially to the geometrical form of the anatomical structure of interest and configured to fixate the anatomical structure of interest in the defined position, when the second curved surface is positioned against the anatomical structure of interest and the fixating element is mounted on the positioning device establishing the detachable connection between the positioning device and the fixating element.

Embodiments may have the beneficial effect of ensure that the anatomical structure of interest is positioned is position in the medical radiation delivery system in the same position as in the magnetic resonance imaging system for MRI data acquisition.

According to embodiments, the method further comprises the acquiring of the magnetic resonance imaging data of the anatomical structure of interest, when the anatomical structure of interest is position in the defined position against the curved antenna surface. The anatomical structure of interest is fixated in the defined position against the antenna element during data acquisition using the fixating element detachably mounted on the antenna element. Since the position is defined by the antenna element of the MRI system, the position may be optimized for acquiring high quality MRI data.

In another aspect, the invention relates to a method for generating a fixating element for fixating an anatomical structure of interest of a subject positioned in a position against a curved antenna surface of an antenna element of a magnetic resonance imaging system defined for acquiring magnetic resonance imaging data of the anatomical structure of interest and in the same position against a first curved surface of a positioning device of a medical radiation delivery system. The method comprises adapting the fixating element to a geometrical form of the anatomical structure of interest in order to generate a second curved surface of the fixating element. The second curved surface is configured to fixate the anatomical structure of interest in the defined position, when the second curved surface is positioned against the anatomical structure of interest and the anatomical structure of interest is positioned against one of the following: the curved antenna surface or the first curved.

Embodiments may have the beneficial effect that the curved surface of the fixating element may efficiently be adapted to the geometrical structure of the anatomical structure of interest. The adaption may either be executed, when the anatomical structure of interest is positioned against the curved surface of the positioning device or when the anatomical structure of interest is positioned against the curved surface of the antenna element. Thus, a fixating element may be generated for the positioning device and in addition used for fixating the anatomical structure of interest in the predefined position at the antenna element or the fixating element may be generated for the antenna element and in addition be used for fixating the anatomical structure of interest in the predefined position at the positioning device.

Embodiments may have the beneficial effect that independent of the precise form and shape of the curved surface of the fixating element, the alignment means may always be arranged at the same positions defined by the receptions. Thus, the alignment may be independent of the precise shape and dimensions of the curved surface.

The anatomical structure of interest may either be positioned on the curved antenna surface, i.e. on the antenna element of the magnetic resonance imaging system, or on the first curved surface of the positioning device of the medical radiation delivery system. The generated fixating element may be used for fixating the anatomical structure of interest on the antenna element as well as on the positioning device.

According to embodiments, the generating further comprises rigidly attaching alignment means to the fixating element for aligning the fixating element on the antenna element as well as on the positioning device. The alignment means are provided in receptions configured for receiving the alignment means. The receptions are comprised by one of the following: the antenna element or the positioning device.

Embodiments may have the beneficial effect that the generation of the fixating element may result in an aligning and mounting of the fixating element on the antenna element or the positioning device.

According to embodiments, the alignment means in addition is configured for mounting the fixating element on the antenna element as well as on the positioning device. In other words, the alignment means may provide mounting means for mounting the fixating elements on the antenna element as well as on the positioning device.

For example, alignment holes may be created in the posterior part of a MRI head-coil. Single-use plastic pins may be inserted into the alignment holes and a flexible net structure may cast over the anatomical structure of interest placed on the posterior part as well as over the pins. After hardening of the mask during MR imaging, the MRI mask with the pins rigidly fixed to the same may be taken out of the MRI head-coil. For usage in a medical radiation delivery system, the MRI mask may be mounted on the positioning device, thus transforming the MRI mask into an RT mask. The positioning device may comprise alignment holes fitted to the alignment pins of the MRI mask as well as mounting means, like e.g. screws, for mounting the positioning device together with the fixating element on a support of the medical radiation delivery system.

According to embodiments, the adapting comprises fitting a flexible pre-form of the fixating element to the anatomical structure of interest positioned against one of the following: the curved antenna surface or the first curved surface. The adapting further comprises hardening the fitted pre-form.

For example, after a subject's head has been positioned on a posterior part of an MRI head-coil or on a positing device, a flexible net structure may be cast over the subject's face as well as tightly into a specific shape of the posterior part of the MRI head-coil or positing device and over upper ends of alignment pins located in predefined alignment holes of the MRI head-coil or positing device. The specific shape of the posterior part of the MRI head-coil and the positing device may be equal. The net structure may be cooled down and thereby hardened. Hardening of the mask during cooling down may typically take up to e.g. 15 min. This time may already in case of the MRI head-coil be used for MRI, which requires the patient to lie still as well. This workflow reduces the overall time for the patient to lie still. The hardened mask rigidly fixed to the alignment pins may be removed from the MRI head-coil afterwards.

The fixating element used for MRI, e.g. a MRI mask, may be complemented with the positioning device basically transforming the MRI mask into an RT mask. The positioning device may comprise alignment holes fitted to the alignment pins of the MRI mask and the positioning device may in addition provided with mounting means, like e.g. screws, for mounting the fixation positioning device on a support of a medical radiation delivery system. If the positioning device is fixed to the MRI mask in a removable way, it may be removed later, and the MRI mask may be reused for MRI-based monitoring or for MRI to update radiation therapy simulation for future radiation delivery. Embodiments may have the beneficial effect that the positioning device may be produced as a standard device with the same standardized shape and dimensions for each subject, because it basically replaces the mechanics of an antenna element, like e.g. an MRI head-coil.

According to embodiments, the positioning device may be an open configuration, i.e. the second curved surface may not be covered by the positioning device, allowing to place e.g. a flat mask pre-form over the anatomical structure of interest to generate a mask. The mask may as well be easily positioned over the anatomical structure of interest. The flat mask pre-form may e.g. be heated and stretched over the anatomical structure of interest. According to embodiments, the antenna element may have an open configuration as well, i.e. the curved antenna surface may not be covered by the antenna element, or a portion of the antenna element covering the curved antenna surface may be detachable for placing a flat mask pre-form or a mask over the anatomical structure of interest.

According to embodiments, the fitted pre-form is hardened during acquisition of magnetic resonance data using a magnetic resonance imaging system.

According to embodiments, the adapting comprises acquiring geometrical data identifying at least partially the geometrical form of the anatomical structure of interest positioned against one of the following: the curved antenna surface or the first curved surface. The generating further comprises 3D-printing of the fixating element comprising the second curved surface defined using the geometrical data.

According to embodiments, the geometrical data may be acquired by means of laser scanning.

According to embodiments, the geometrical data may be acquired from an additional fixating element, like e.g. an additional face mask, and provided in form of a positive model of at least part of the anatomical structure generated using the additional fixating element. For example, the additional fixating element may provide an additional second surface at least partially adapted to the geometrical form of the anatomical form of interest which is used as a negative model of at least part of the anatomical structure.

For example, a fixating element in form of an MRI mask may be scanned by a laser scanner. The known shape of the antenna element and/or the positions of the alignment pins may be recognized by rigid body registration to the scanned form of the MRI mask and/or point set of alignment pin positions. A computer may then complement the scanned form of the MRI mask to generate the RT mask equipped with alignment means, like e.g. pins. In addition, the position device comprising mounting elements, like e.g. screws, for alignment and fixation on the support of the medical radiation delivery system may be designed in-silico. Finally, the RT mask and/or the positioning device may be produced using 3 D-printing.

The above process fits well with the workflow of first performing the MRI for radiation therapy simulation and positioning the anatomical structure of interest for radiation delivery. However, the reverse process may be possible as well: a fixating element for the positioning device, i.e. an RT mask may be produced first, and then may be used to produce a fixating element for the antenna element, i.e. an MRI mask.

Alternatively, a mechanic process maybe used: the RT mask may e.g. be filled with some flexible material, like e.g. first a plastic foil, and then construction foam, that hardens to provide a positive copy of the anatomical structure of interest. This positive copy may be fitted into the positioning device including the alignment holes. Alignment means, like e.g. single-use plastic pins, may be applied to the holes and a flexible net structure may cast over the positive copy as well as the pins. After hardening of the net, the MRI mask including the pins may be removed and used during MRI.

The aforementioned embodiments of the invention may enable a positioning of an anatomical structure of interest in an equal position on an antenna element of an MRI system and on a support of a radiation delivery system. The position is defined by the antenna element and may thus be optimized for MRI data acquisition. In particular, a usage of a dedicated antenna element, like a head-coil is enabled. Furthermore, the fixating element may ensure an efficient fixation of the anatomical structure of interest in the defined position for the MRI data acquisition as well as for radiation delivery. Finally, an efficient method is provided for generating fixating elements customized for individual anatomical structure of interest. Thus, the positioning and/or fixating may be optimized for individual anatomical structure, like e.g. a face and/or head, while the antenna element and/or the positioning device may be designed based on general geometrical features of a specific type of anatomical structures of interest, like e.g. a head.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium maybe referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium maybe transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code maybe in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) imaging data is defined herein as being the recorded measurements of radio frequency signals emitted by atomic spins using the antenna of a magnetic resonance imaging system during a magnetic resonance imaging scan. A Magnetic Resonance Imaging (MRI) image or MR image is defined herein as being the reconstructed two or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 shows an example of an antenna element;
Fig. 2 shows an example of an additional part of the antenna element of Fig. 1;
Fig. 3 shows an example of a fixating element;
Fig. 4 shows an example of an antenna element with alignment means;
Fig. 5 shows an example of a pre-form for a fixating element;
Fig. 6 shows an example of a positioning device;
Fig. 7 shows an example of positioning device with alignment means;
Fig. 8 shows an example of a magnetic resonance imaging system;
Fig. 9 shows an example of a radiation delivery system;
Fig. 10 illustrates an exemplary method of positioning an anatomical structure of interest;
Fig. 11 illustrates an exemplary method of generating a fixating element; and
Fig. 12 illustrates a further exemplary method of generating a fixating element.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

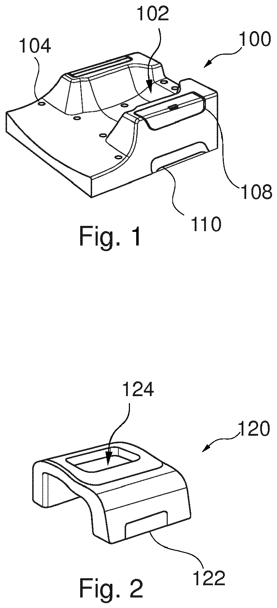

Fig. 1 shows an exemplary antenna element 100, which comprises a curved surface 102 for positioning an anatomical structure of interest. The antenna element 100 shown in Fig. 1 is a head-coil, more precisely a posterior part of a head-coil. The anatomical structure of interest, like e.g. a head, is placed on the curved surface 102, thus forcing the anatomical structure of interest into a position defined by the antenna element 100. The curved surface 102 against which the anatomical structure of interest is positioned ensures that the distance between antenna element 100 and anatomical structure of interest is minimized. In addition, since the position in which the MRI data is acquired is defined by the curved surface 102 using an equal curved surface, the respective position defined by the antenna element 100 may be mimicked, when positioning the anatomical structure of interest in other system, like e.g. a medical radiation delivery system or a CT imaging system. Furthermore, the antenna element 100 is shown to comprise mounting means 110 configured for mounting the antenna element 100 in an MRI system, e.g. on a support of an MRI system. In addition, the antenna element 100 may comprise mounting means 108 configured for mounting an additional part 120 of the antenna element 100, like the part shown in Fig. 2, on the antenna element 100. The additional part may cover the curved surface 102 as well as the anatomical structure of interest position against the curved surface 102 at least partially.

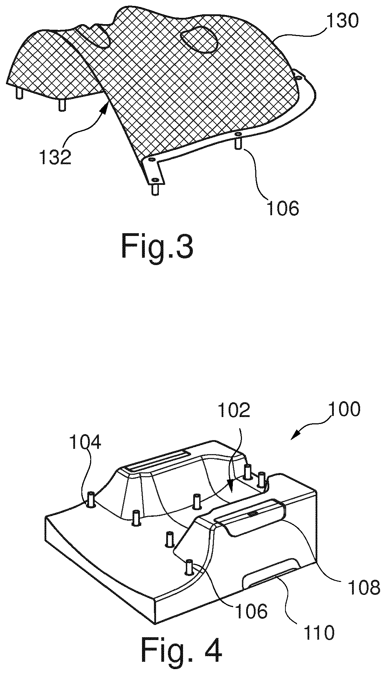

Furthermore, the antenna element 100 may comprise a plurality of receptions 104 configured as mounting means for mounting a fixating element 130, like e.g. the face mask 130 of Fig. 3, onto the antenna element 100. When the anatomical structure of interest is arranged between the curved surface 102 and an additional curved surface provided by the fixating element 130, the anatomical structure of interest maybe fixated in a position defined by the antenna element 100. In the receptions 104 alignment means 106, like e.g. pins, of the fixating element 130 maybe insert. The alignment means 106 may align the fixating element 130 on the antenna element 100. In addition, alignment means 106 may be used to mount the fixating element 130 on the antenna element 100, when being insert in the receptions 104.

Fig. 2 shows an additional part 120 for the antenna element 100 configured to be mounted on the antenna element 100 using mounting means 122. The additional part 120 is configured to cover the face of a head positioned with the back of the head against the curved surface 102. Thus, the antenna element 100 and the additional part 120 may in combination provide a separatable head-coil. The head-coil may comprise a plurality of coil elements, wherein the antenna element 100 may comprise one or more posterior coil elements and the additional part 120 may comprise one or more anterior coil elements. For positioning the anatomical structure of interest on the antenna element 100, the additional part 120 may be dismounted from the antenna element 100, the anatomical structure of interest may be positioned against the curved surface 102 and the anatomical structure of interest may be fixated in the position defined by the antenna element 100 by mounting the fixating element 130 on the antenna element 100. When the anatomical structure of interest is fixated in the respective position, additional part 120 maybe mounted on the antenna element 100 covering the anatomical structure of interest as well as the fixating element 130. The additional part 120 may e.g. comprise a clearance 124, into which or through which part of the anatomical structure of interest and/or the fixating element 130 may extend. Thus, coil elements comprised by the additional part 120 may be positioned as close as possible to the anatomical structure of interest without coming into conflict with the fixating element 130.

Fig. 3 shows an exemplary fixating element 130 which comprises a curved surface 132, which may be provided by a net structure and customized for an individual anatomical structure of a subject in order to fixate an individual anatomical structure in the predefined position, when the anatomical structure of interest is positioned against the curved surface 132. The fixating element 130 is provided in form of a face mask, which maybe adapted to the individual facial form of an individual subject. For the purpose of fixating, the fixating element 130 may comprise a plurality of alignment means 106, which are configured to be placed in the receptions 104 provided by the antenna element 100. While the fixating element 130 is customized, the antenna element 100 and the additional part 120 may be used for a plurality of different subjects. In order to ensure an optimal positioning and fixating, an individual fixating element 130 may be used for each of the subjects adapted to specific geometrical features of the anatomical structure, e.g. face, of the respective subject.

In order to generate a customized fixating element 130 as shown in Fig. 3, alignment means 106, e.g. in form of pins, may be positioned in the receptions 104 of the antenna element 100 of Fig. 1 as shown in Fig. 4.

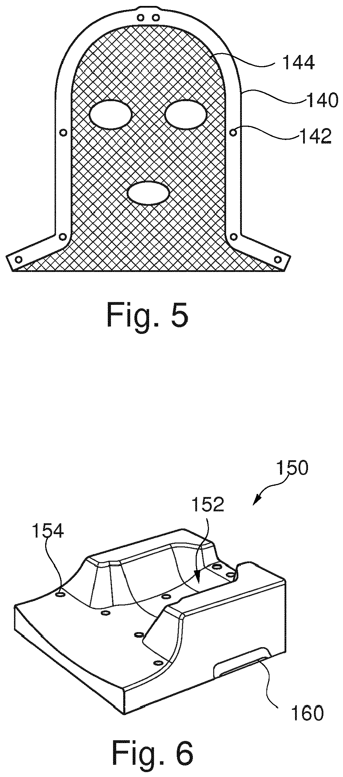

Fig. 5 shows an exemplary perform 140 for generating the fixating element 130 of Fig. 3. The pre-form may comprise a flexible net structure 144 which is cast over the anatomical structure of interest, e.g. the face of a head positioned on the curved surface 102 of the antenna element 100 of Fig. 4. Thus, the pre-form 130 may be fitted to the individual features of the geometrical form of an individual anatomical structure of interest, like a face and a head form. In addition, the pre-form 140 may comprise receptions 142 which may be cast over the alignment means 106 located at the antenna element 100. The fitted pre-form may be hardened in order to generate a rigid curved surface adapted to the individual geometrical features resulting in the fixating element 130 of Fig. 3. Furthermore, due to the hardening, the fitted pre-form 130 may be rigidly connected to the alignment means 106. Thus, it maybe ensured that independently of the precise form and dimension of the curved surface 122, the resulting fixating element 130 may always be aligned with the antenna element 100.

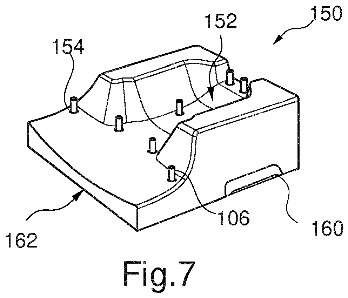

Fig. 6 shows a positioning device 150. The positioning device 150 comprises a curved surface 152, is equal to the curved surface 102 of the antenna element 100 of Fig. 1. Thus, an anatomical structure of interest, e.g. a head, positioned against the curved surface 152 of the positioning device 150 is forced into a position equal to the position defined by the antenna element 100. For this purpose, the curved surface 152 maybe adapted to general geometrical features of a particular type of anatomical structures, like e.g. heads, necks, knees, or shoulders. The positioning device may further comprise receptions 154, i.e. mounting means, for receiving alignment means 106 of a fixating element 130 as shown in Fig. 7. The fixating element 130 maybe designed to fixate the anatomical structure of interest in the position according to the curved surface 152 of the positioning device 150 and in particular to prevent a twisting within or removing from the respective position. Furthermore, the positioning device 150 comprises further mountings means 160 for mounting the positioning device 150 onto a support of a radiation delivery system. For example, the positioning device 150 may comprise a flat bottom face to be positioned on a standard support in form of a flat tabletop. According the embodiments, the positioning device 150 is radiolucent for radiation delivered by the radiation delivery system for which it is configured, e.g. radiolucent for X-rays and/or gamma radiation.

Fig. 7 shows the positioning device 150 of Fig. 6 with alignment means 106 located in the receptions 154. Thus, the fixating element 130 of Fig. 3 may alternatively be generated by casting the pre-form 140 shown in Fig. 5 over an anatomical structure of interest, like e.g. a head positioned with its back on the curved surface 152, as described above with the antenna element 100 replaced by the positioning device 150.

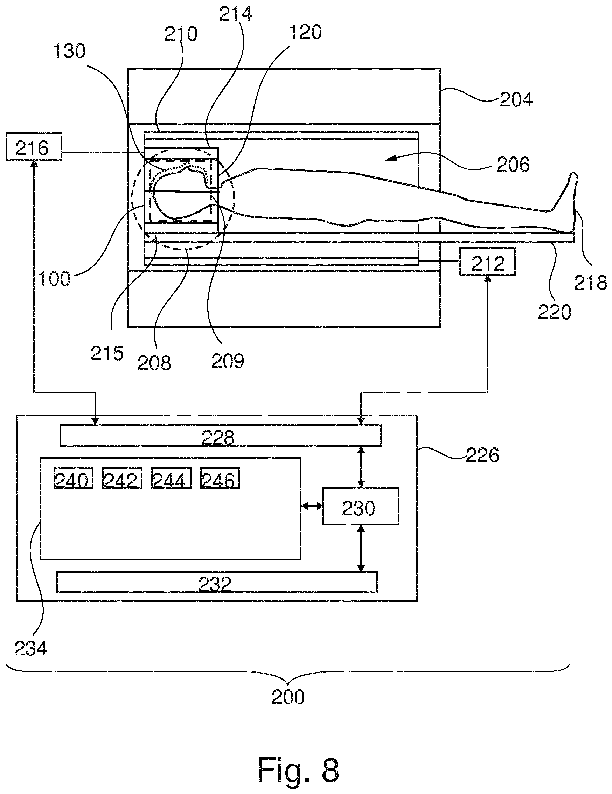

Fig. 8 shows an example of a magnetic resonance imaging system 200 with a magnet 204. The magnet 204 is a superconducting cylindrical type magnet with a bore 206 through it. The use of different types of magnets is also possible. For instance, it is also possible to use both a split cylindrical magnet and a so called open magnet. A split cylindrical magnet is similar to a standard cylindrical magnet, except that the cryostat has been split into two sections to allow access to the axial plane through the iso-center of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 206 of the cylindrical magnet 204 there is an imaging zone 208 where the magnetic field is strong and uniform enough to perform magnetic resonance imaging. A region of interest 209 is shown within the imaging zone 208. The magnetic resonance data is typically acquired for the region of interest. A subject 218 is shown as being supported by a subject support 220 such that at least a portion of the subject 218 comprising an anatomical structure of interest, like e.g. a head, is within the imaging zone 208 and the region of interest 209.

Within the bore 206 of the magnet there is also a set of magnetic field gradient coils 210 which is used for acquisition of magnetic resonance data to spatially encode magnetic spins within the imaging zone 208 of the magnet 204. The magnetic field gradient coils 210 are connected to a magnetic field gradient coil power supply 212. The magnetic field gradient coils 210 are intended to be representative. Typically, magnetic field gradient coils 210 contain three separate sets of coils for spatially encoding in three orthogonal spatial directions. A magnetic field gradient power supply supplies current to the magnetic field gradient coils. The current supplied to the magnetic field gradient coils 210 is controlled as a function of time and may be ramped or pulsed.

Adjacent to the imaging zone 208 a head-coil is arranged comprising an antenna element 100 according to Fig.1 as well as an additional part 120 according to Fig.2. The position of the anatomical structure of interest, i.e. the head, is fixated against the antenna element 100 by a fixating element 130, like e.g. a face mask, of Fig. 3. The head-coil comprises a plurality of radio-frequency coils 214, 215 for manipulating the orientations of magnetic spins within the imaging zone 208 and for receiving radio transmissions from spins also within the imaging zone 208. The radio frequency antenna 100, 120 may contain multiple coils and/or coil elements. The radio frequency antenna 100, 120 may also be referred to as a channel or antenna. The radio-frequency coils 214, 215 are connected to a radio frequency transceiver 216. The radio-frequency coils 214, 215 and radio frequency transceiver 216 may be replaced by separate transmit and receive coils and a separate transmitter and receiver. It is understood that the radio-frequency coils 214, 215 and the radio frequency transceiver 216 are representative. The radio-frequency coils 214, 215 are intended to also represent a dedicated transmit antenna and a dedicated receive antenna. Likewise, the transceiver 216 may also represent a separate transmitter and separate receivers. The radio-frequency coils 214, 215 may also have multiple receive/transmit elements and the radio frequency transceiver 216 may have multiple receive/transmit channels. For example, if a parallel imaging technique such as SENSE is performed, the radio-frequency coils 214, 215 will have multiple coil elements.

The transceiver 216 and the gradient controller 212 are shown as being connected to a hardware interface 228 of a computer system 226. The computer system further comprises a processor 230 that is in communication with the hardware system 228, a memory 234, and a user interface 232. The memory 234 may be any combination of memory which is accessible to the processor 230. This may include such things as main memory, cached memory, and also non-volatile memory such as flash RAM, hard drives, or other storage devices. In some examples the memory 234 may be considered to be a non-transitory computer-readable medium.

The memory 234 is shown as containing machine-executable instructions 240. The machine-executable instructions 240 enable the processor 230 to control the operation and function of the magnetic resonance imaging system 200. The machine-executable instructions 240 may also enable the processor 230 to perform various data analysis and calculation functions. The computer memory 234 is further shown as containing pulse sequence commands 242.

The pulse sequence commands 242 are configured for controlling the magnetic resonance imaging system 200 to acquire magnetic resonance data 244 from the region of interest 209. Using the acquire magnetic resonance data 244 magnetic resonance images 246 are reconstructed. The reconstructed images 246 be displayed on a display provided by the user interface 232. Furthermore, the reconstructed images 246 may e.g. be used for a radiation simulation and/or generating a dose plan.

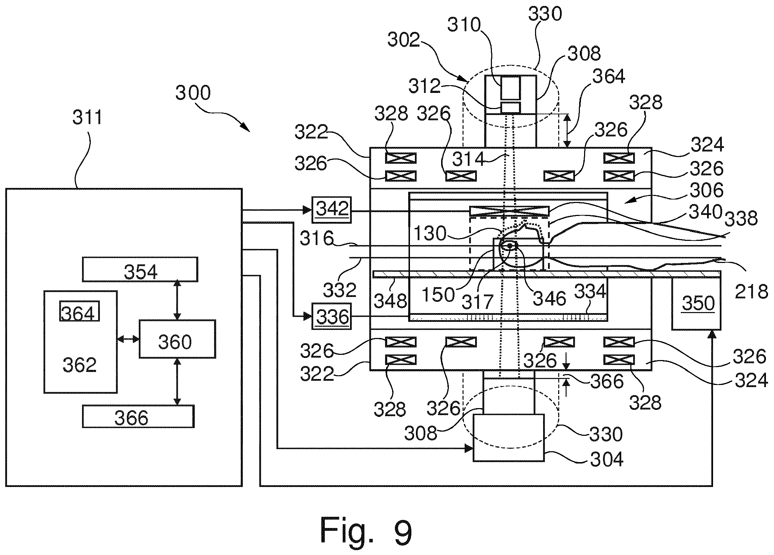

Fig. 9 shows a cross-sectional view of an exemplary medical radiation delivery system. The example of Fig. 9 is a magnetic resonance image-guided system. The system 300 is shown as comprising a radiation delivery module 302 and a magnetic resonance imaging module 306. The radiation delivery module 302 comprises a ring mechanism 308. The ring mechanism 308 supports a radiation source 310. The radiation source 310 is representative and may e.g. be a LINAC x-ray source or and a radioisotope gamma radiation source. Adjacent to the radiation source 310 is a multi-leaf beam collimator 312 for collimating a radiation beam 314 that is generated by the radiation source 310. The ring mechanism 308 is also adapted for moving e.g. rotating the radiation source 310 and the beam collimator 312 about a rotational point 317 of the radiation delivery module 302. A rotational axis 316 passes through the rotational point 317.

The magnetic resonance imaging module 306 is shown as comprising a main magnet 322. The ring mechanism 308 is ring-shaped and surrounds the main magnet 322. The main magnet 322 shown in Fig. 9 is a cylindrical type superconducting magnet. However, other magnets are also applicable for embodiments of the invention. The main magnet 322 has a supercooled cryostat 324. Inside the cryostat 324 there is a collection of superconducting coils 326. There are also compensation coils 328 whose current opposes the direction of current in the superconducting coils 326. This creates a low magnetic field zone 330 that circles or encompasses the main magnet 322. The cylindrical main magnet 322 is shown as having an axis 332 of symmetry.

Within the bore of the magnet there is a magnetic field gradient coil 334 which is used for acquisition of image magnetic resonance data to spatially encode objects within an imaging volume 338 of the main magnet 322. The magnetic field gradient coil 334 is connected to a magnetic field gradient coil power supply 336. The magnetic field gradient coil 334 is intended to be representative. Typically, magnetic field gradient coils contain three separate sets of coils for spatially encoding in three orthogonal spatial directions. The imaging volume 338 is located in the center of the main magnet 322.

Adjacent to the imaging volume 338 is a radio frequency (RF) coil 340 for manipulating the orientations of magnetic spins within the imaging volume 338 and for receiving radio transmissions from spins also within the imaging volume 338. The radio frequency coil 340 is connected to a radio frequency transceiver 342. The radio frequency coil 340 and radio frequency transceiver 342 may be replaced by separate transmit and receive coils and a separate transmitter and receiver. It is understood that the radio frequency coil 340 and the radio frequency transceiver 342 are simply representative.

Within the center of the main magnet 322 is also located a subject 218. The subject 218 has a target volume (or target zone) 346 and is shown as reposing on a support 348. The RF coil 340 may transmit RF pulses into the target volume 346. The support 348 has a mechanical positioning system 350. The mechanical positioning system 350 is adapted for positioning the support 348 within the main magnet 322. Depending upon the space available inside of the main magnet 322, the mechanical positioning system 350 may move the support 348 in different directions including a direction perpendicular to the magnet axis 332. If there is more space available inside the main magnet 322 the mechanical positioning system 350 may have more degrees of freedom. For instance, the mechanical positioning system 350 may position the support 348 with six degrees of freedom.

The anatomical structure of interest comprising the target volume (or target zone) 346, e.g. a head, is positioned relative to the support 348 in form of a flat tabletop using a positioning device 150, as shown in Fig. 6. The positioning device 150 is mounted on the support 348. The head is fixated against the positioning device 150 by a fixating element 130, e.g. in form of a face mask as shown in Fig. 3. The positioning device 150 maybe configured to position the anatomical structure of interest in the same position as defined by the antenna element 100 in Fig. 8. For a precise positioning, the positioning device 150 and/or the fixating element 130 may be provided with laser markers.

The radio frequency transceiver 342, the magnetic field gradient coil power supply 336, the mechanical actuator 304, and the mechanical positioning system 350 are all shown as being connected to a hardware interface 354 of a computer 311 configured for controlling the radiation delivery system 300.

The radiation delivery module 302 maybe, for example, connected to the hardware interface 354 and communicates with the computer 311 via the mechanical actuator 304 or via other means enabling communication between radiation delivery module 302 and the computer 311.

The computer 311 may comprise a processor 360 for controlling the radiation delivery system 300 by executing machine readable instructions 364 stored in a memory 362 of the computer 311. Furthermore, the computer 311 may comprise a user interface 366.

For the example shown in Fig. 9, the rotational axis 316 of the radiation delivery module is not coaxial with the magnet axis 332. The rotational point 317 is shown as being off center from the magnet axis 332. It can be seen that the target zone 346 is off-center and away from the magnet axis 332. The radiation delivery module 302 has been moved by mechanical actuator 304 such that the rotational point 317 of the radiation delivery module is within the target zone 346. It can be seen that the ring mechanism 308 has been moved relative to the magnet 322.

The radiation beam 314 passes through the rotational point 317. Placing the rotational point 317 at the center of the target zone 346 allows the target zone to be treated continuously when the radiation beam 314 is created by the radiation source 310 and is rotated by the ring mechanism 308.

According to embodiments, the computer 311 may receive reconstructed magnetic resonance images 246 from a magnetic resonace imaging system, like the magnetic resonace imaging system of Fig. 8, used for radiation simulation. The magnetic resonance images 246 may be used for defining the position in which the subject 218 is to be positioned.

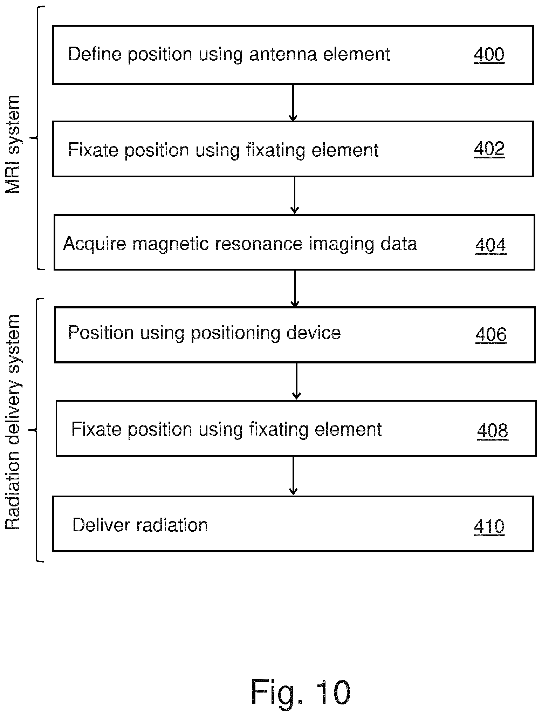

Fig. 10 shows a flowchart which illustrates an exemplary method of positioning an anatomical structure of interest of a subject on a support of a medical radiation delivery system according to a position defined by an antenna element of a magnetic resonance imaging system. In step 400, the position is defined by the antenna element comprising a curved surface against which the anatomical structure of interest is positioned for acquiring magnetic resonance images. In step 402, the anatomical structure of interest is fixated in the position defined by the antenna element using a fixating element such that the anatomical structure of interest is prevented from changing ist orientation and/or being moved relative to the positioning device. The fixating element is mounted on the antenna element such that it at least partially covers the anatomical structure of interest. In step 404, magnetic resonance imaging data of the anatomical structure in the position defined by the antenna element is acquired. The acquired magnetic resonance imaging data may e.g. be used for a radiation delivery simulation and planning of radiation doses to be delivered to the anatomical structure of interest using a radiation delivery system.

In step 406, the anatomical structure of interest is positioned on the support of the medical radiation delivery system according to a position defined by the antenna element of the magnetic resonance imaging system in step 400. The anatomical structure of interest is positioned against a curved surface of a positiong device, which is mounted on the support. The curved surface of the positiong device is equal to the curved surface of the antenna element. The antenna element may be comprised by a support or mounted on a support of the magnetic resonance imageing system. Thus, a flat tabletop support may be used by the radiation delivery system for supporting the subject, while the anatomical structure of interest may nevertheless be positioned in the same position as for the magnetic data acquisition, when being positioned against the curved suface of the positioning device. In step 408, the anatomical structure of interest is fixated against the curved surface of the positioning device in the position defined by the antenna element using a fixating element monted on the positioning device. When the anatomical structure of interest is fixated in the same position for which the radiation delivery has been simultaed and for which the doses have been planned, in step 410 radiation may be delivered.

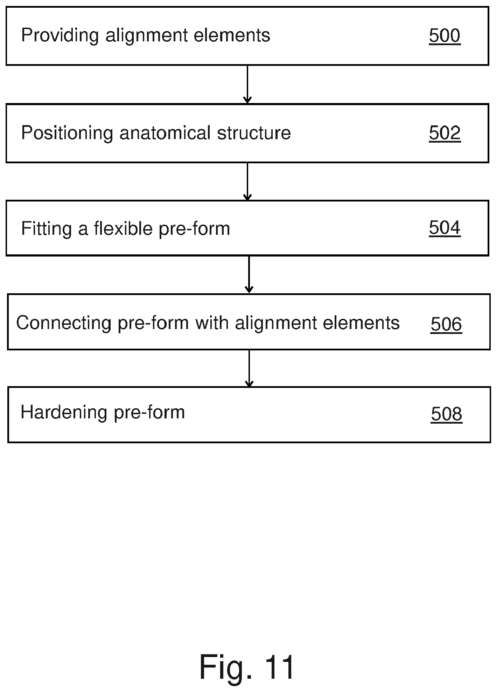

Fig. 11 shows a flowchart which illustrates an exemplary method of generating a fixating element for an antenna element and/or a positioning device. In step 500, either the antenna element or the positioning device is provided with alignment means for the fixating element in order to enable an alignment of the fixating element on the antenna element and/or the positioning device. In step 502, the anatomical structure of interest is position either against a curved surface of the antenna element or the positioning device. In step 504, a flexible pre-form of the fixating element, e.g. a flexible net structure, is cast over the anatomical structure of interest positioned in a defined by the curved surface either on the antenna element or on the positioning device. In step 506, the flexible pre-form is further casted over the alignment means and connected with the same. In step 508, the fixating element is hardened such that a rigid connection is formed with the fixating means. In case the fixating element is generated using the positioning device, the fixating element may just be generated for usage in the radiation delivery system after it is hardened. Alternatively, it may first be generated without delivering radiation and provided to magnetic resonance imaging system. In the magnetic resonance imaging system, it may be used and afterwards it may in addition be used in the radiation delivery system. In case the fixating element is generated using the antenna element, the hardening time may be used for acquiring magnetic resonance data. After the fixating element is hardened and the magnetic resonance imaging data has been acquired, the fixating element may be provided to the radiation delivery system. Alternatively, the generated fixating element may only be used for the magnetic resonance imaging data acquisition in the magnetic resonance imaging system.

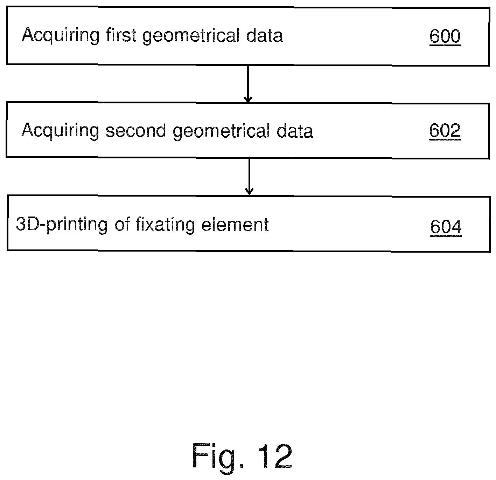

Fig. 12 shows a flowchart which illustrates a further exemplary method of generating a fixating element. In step 600, first geometrical data is acquired. The first geometrical data may comprise geometrical data of an antenna element or a positioning device. For example, the first geometrical data may define a section of the antenna element and/or a positioning device on which the fixating element is to be mounted. For example, geometrical data regarding location and form of receptions for receiving alignment means for aligning the fixating element may be comprised. The first geometrical data may for example be acquired using laser scanning. In step 602, second geometrical data may be acquired. The second geometrical data may define the geometrical form of an anatomical structure of interest to be fixated by the fixating means. The second geometrical data may for example be acquired for the anatomical structure of interest positioned against a curved surface of an antenna element or a positioning device. Alternatively, a model of the anatomical structure of interest positioned against a curved surface of an antenna element or a positioning device may be used. The model may either be a positive or a negative form of the anatomical structure of interest. For example, the model may be a further fixating element already generated for the respective anatomical structure of interest, e.g. using the method of Fig. 11. The second geometrical data may for example be acquired using laser scanning. In step 602, the fixating element is generated, e.g. using 3D-printing, according to the first and the second geometrical data. Thus, a fixating element may be customized for an individual anatomical structure of interest as well as an antenna element and/or a positioning device.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: antenna element
- 102: curved surface
- 104: reception
- 106: alignment means
- 108: mounting means
- 110: mounting means
- 120: additional part
- 122: mountig means
- 124: clearance
- 130: fixating element
- 132: curved surface
- 140: pre-form
- 142: reception
- 144: flexible net structure
- 150: positioning device
- 152: curved surface
- 154: reception
- 160: mounting means
- 162: bottom face
- 200: magnetic resonance imaging system
- 204: magnet
- 206: bore of magnet
- 208: imaging zone
- 209: region of interest
- 210: magnetic field gradient coils
- 212: magnetic field gradient coil power supply
- 214: radio-frequency coil
- 215: radio-frequency coil
- 216: transceiver
- 218: subject
- 220: subject support
- 226: computer system
- 228: hardware interface
- 230: processor
- 232: user interface
- 234: computer memory
- 240: machine executable instructions
- 242: pulse sequence commands
- 244: magnetic resonance imaging data
- 246: magnetic resonance image
- 300: radiation delivery system
- 302: radiation delivery module
- 304: mechanical actuator
- 306: magnetic resonance imaging module
- 308: ring mechanism
- 310: radiation source
- 311: computer
- 312: multi-leaf beam collimator
- 314: radiation beam
- 316: rotational axis
- 317: rotational point
- 322: main magnet
- 324: cryostat
- 326: superconducting coil
- 328: compensation coil
- 330: low magnetic field zone
- 332: magnet axis
- 334: magnetic field gradient coil
- 336: magnetic field gradient coil power supply
- 338: imaging volume
- 340: radio frequency coil
- 342: radio frequency transceiver
- 218: subject
- 346: target volume
- 348: support
- 350: mechanical positioning system
- 354: hardware interface
- 360: processor
- 362: memory
- 364: machine readable instructions
- 366: user interface

## Claims

1. An antenna element (100) for a magnetic resonance imaging system (200), the antenna element (100) being configured for acquiring magnetic resonance imaging data (244) of an anatomical structure of interest of a subject (218),
wherein the antenna element (100) comprises a curved antenna surface (102), wherein the curved antenna surface (102) is adapted at least partially to a geometrical form of the anatomical structure of interest and configured to force the anatomical structure of interest in a position defined by the antenna element (100) for acquiring the magnetic resonance imaging data, when the anatomical structure of interest is positioned against the curved antenna surface (102),
wherein the antenna element (100) further comprises mounting means (104) configured for mounting a fixating element (130) on the antenna element (100) and establishing a detachable connection between the antenna element (100) and the fixating element (130),
wherein the fixating element (130) comprises a second curved surface (132), wherein the second curved surface (132) is adapted at least partially to the geometrical form of the anatomical structure of interest and configured to fixate the anatomical structure of interest in the defined position, when the second curved surface (132) is positioned against the anatomical structure of interest and the fixating element (130) is mounted on the antenna element (100) establishing the detachable connection between the antenna element (100) and the fixating element (130).

2. The antenna element (100) of claim 1, wherein the geometrical form of the anatomical structure of interest to which the curved antenna surface (102) is adapted is defined by general geometrical features common to a plurality of anatomical structures of a plurality of subjects (218) that are of the same type as the anatomical structure of interest.

3. The antenna element (100) of any of claims 1 and 2, wherein the fixating element (130) is mounted on the antenna element (100).

4. The antenna element (100) of claim 3, wherein the geometrical form of the anatomical structure of interest to which the second curved surface (132) is adapted is defined by individual geometrical features of the anatomical structure of interest of the subject (218).

5. The antenna element (100) of any of claims 3 and 4, wherein the fixating element (130) comprises a net structure, the net structure being configured to at least partially covering the anatomical structure of interest and to provide the second curved surface (132).

6. A positioning device (150) for positioning an anatomical structure of interest of a subject (218) on a support (348) of a medical radiation delivery system (300) according to a position defined by means of magnetic resonance imaging data (244) of the anatomical structure of interest acquired using an antenna element (100) of a magnetic resonance imaging system (200),
wherein the positioning device (150) comprises a first curved surface (152) equal to an antenna surface (102) of the antenna element (100), wherein the first curved surface (152) is adapted at least partially to a geometrical form of the anatomical structure of interest and configured to force the anatomical structure of interest in the defined position, when the anatomical structure of interest is positioned against the first curved surface (152),
wherein the positioning device (150) further comprises first mounting means (160) configured for mounting the positioning device (150) on the support (348),
wherein the positioning device (150) further comprises second mounting means (154) configured for mounting a fixating element (130) on the positioning device (150) and establishing a detachable connection between the positioning device (150) and the fixating element (130),
wherein the fixating element (130) comprises a second curved surface (132), wherein the second curved surface (132) is adapted at least partially to the geometrical form of the anatomical structure of interest and configured to fixate the anatomical structure of interest in the defined position, when the second curved surface (132) is positioned against the anatomical structure of interest and the fixating element (130) is mounted on the positioning device (150) establishing the detachable connection between the positioning device (150) and the fixating element (130), and
wherein the positioning device (150) is radiolucent for radiation delivered by the medical radiation delivery system (300).

7. The positioning device (150) of claim 6, wherein the fixating element (130) is mounted on the positioning device (150).

8. A fixating element (130) for fixating an anatomical structure of interest of a subject (218) positioned in a position against a curved antenna surface (102) of an antenna element (100) of a magnetic resonance imaging system (200)defined for acquiring magnetic resonance imaging data (244) of the anatomical structure of interest and in the same position against a first curved surface (152) of a positioning device (150) of a medical radiation delivery system (300), wherein the fixating element (130) comprises mounting means (106) configured for mounting the fixating element (130) on the antenna element (100) as well as on the positioning device (150) and establishing a detachable connection between the fixating element (130) and the antenna element (100) as well as between the fixating element (130) and the positioning device (150), wherein the fixating element (130) further comprises a second curved surface (132) adapted at least partially to the geometrical form of the anatomical structure of interest and configured to fixate the anatomical structure of interest in the defined position, when the second curved surface (132) is positioned against the anatomical structure of interest and the fixating element (130) is mounted on one of the following: the antenna element (100) or the positioning device (150).

9. A kit comprising the antenna element (100) of any of claims 1 to 5 for a magnetic resonance imaging system (200), wherein the antenna element (100) is configured for positioning the anatomical structure of interest of a subject (218) in a position defined by the antenna element (100) for acquiring magnetic resonance imaging data (244) of the anatomical structure of interest and for acquiring the magnetic resonance imaging data,
wherein the kit further comprises the positioning device (150) of any of claims 6 and 7 for positioning the anatomical structure of interest on a support (348) of a medical radiation delivery system (300) according to the position defined by the antenna element (100), and
wherein the kit further comprises the fixating element (130) of claim 8 for fixating the anatomical structure of interest in the defined position against the antenna element (100) as well as against the positioning device (150).

10. A method for positioning an anatomical structure of interest of a subject (218) on a support (348) of a medical radiation delivery system (300) according to a position defined by an antenna element (100) of a magnetic resonance imaging system (200), the method comprising:
- defining the position using the antenna element (100) for acquiring magnetic resonance imaging data (244) of the anatomical structure of interest, wherein the anatomical structure of interest is positioned against a curved antenna surface (102) of the antenna element (100) during data acquisition, the curved antenna surface (102) being at least partially adapted to a geometrical form of the anatomical structure of interest and configured to force the anatomical structure of interest in the defined position, when the anatomical structure of interest is positioned against the curved antenna surface (102),
- positioning the anatomical structure of interest on the support (348) of the medical radiation delivery system (300) according to the position defined by the antenna element (100) using a positioning device (150), wherein the anatomical structure of interest is positioned against a first curved surface (152) of the positioning device (150) which is equal to the curved antenna surface (102),
- fixating the anatomical structure of interest in the defined position against the positioning device (150) using a fixating element (130), wherein the fixating element (130) comprising a second curved surface (132) is mounted on the positioning device (150) establishing a detachable connection between the positioning device (150) and the fixating element (130), wherein the second curved surface (132) is adapted at least partially to the geometrical form of the anatomical structure of interest and configured to fixate the anatomical structure of interest in the defined position, when the second curved surface (132) is positioned against the anatomical structure of interest and the fixating element (130) is mounted on the positioning device (150) establishing the detachable connection between the positioning device (150) and the fixating element (130).

11. The method of claim 10, wherein the method further comprises the acquiring of the magnetic resonance imaging data (244) of the anatomical structure of interest, when the anatomical structure of interest is position in the defined position against the curved antenna surface (102), and wherein the anatomical structure of interest is fixated in the defined position against the antenna element (100) during data acquisition using the fixating element (130) detachably mounted on the antenna element (100).

12. A method for generating a fixating element (130) for fixating an anatomical structure of interest of a subject (218) positioned in a position against a curved antenna surface (102) of an antenna element (100) of a magnetic resonance imaging system (200) defined for acquiring magnetic resonance imaging data (244) of the anatomical structure of interest and in the same position against a first curved surface (152) of a positioning device (150) of a medical radiation delivery system (300), wherein the method comprises adapting the fixating element (130) to a geometrical form of the anatomical structure of interest in order to generate a second curved surface (132) of the fixating element (130), wherein the second curved surface (132) is configured to fixate the anatomical structure of interest in the defined position, when the second curved surface (132) is positioned against the anatomical structure of interest and the anatomical structure of interest is positioned against one of the following: the curved antenna surface (102) or the first curved (152).

13. The method of claim 12, wherein the generating further comprises rigidly attaching alignment means (106) to the fixating element (130) for aligning the fixating element (130) on the antenna element (100) as well as on the positioning device (150), wherein the alignment means (106) are provided in receptions (104, 154) configured for receiving the alignment means (106), wherein the receptions (104, 154) are comprised by one of the following: the antenna element (100) or the positioning device (150).

14. The method of any of claims 12 and 13, wherein the adapting comprises fitting a flexible pre-form (140) of the fixating element (130) to the anatomical structure of interest positioned against one of the following: the curved antenna surface (102) or the first curved surface (152), and wherein the adapting further comprises hardening the fitted pre-form (140).

15. The method of any of claims 12 and 13, wherein the adapting comprises acquiring geometrical data identifying at least partially the geometrical form of the anatomical structure of interest positioned against one of the following: the curved antenna surface (102) or the first curved surface (152), and wherein the generating further comprises 3D-printing of the fixating element (130) comprising the second curved surface (132) defined using the geometrical data.
